Europäisches Patentamt

European Patent Office

Office européen des brevets

(19)

(11) EP 0 841 076 A2

(12) EUROPEAN PATENT APPLICATION

(43) Date of publication:
13.05.1998 Bulletin 1998/20

(51) Int. Cl.$^6$: A61N 2/02

(21) Application number: 97117679.7

(22) Date of filing: 13.10.1997

(84) Designated Contracting States:
AT BE CH DE DK ES FI FR GB GR IE IT LI LU MC NL PT SE
Designated Extension States:
AL LT LV RO SI

(30) Priority: 07.11.1996 IT MI960736 U

(71) Applicant:
Kalor Thermotechnik GmbH
96515 Sonneberg (DE)

(72) Inventor: Wolfsgruber, Guglielmo
22044 Inverigo (Como) (IT)

(74) Representative: Lecce, Giovanni
UFFICIO BREVETTI CALCIATI S.r.l.
via G. Negri, 10
20123 Milano (IT)

(54) Tub having magnetotherapeutic action

(57) Magneto-therapic action tub, comprising a container (1) from insulating material provided on the external surface with at least a bobbin (2) of superimposed coils (3) of a lead, connected to an electric tap.

## Description

The present invention relates to a magneto-therapic action tub.

More particularly, the invention relates to a tube allowing to perform magnetic massages on the body.

Magneto-therapy is a well-known external therapy used in some sectors of medicine by means of the use of magnetism.

It is based on some purported effects arising from mineral and animal magnetism and has been supported by the Austrian doctor F.A. Mesmer.

The effects arising from magnetism seem to be due to the fact that the pulsation of magnetic field at well determined frequency and frequency cycles causes a magnetisation of human body, i.e., it causes the ionised particles present in the body to move.

The applicant has now found that an easy and effective means for applying magneto-therapy consists in the submission of the subject to a bath in a tub provided on its external surface with a coil of a lead or conductive strip connected to an electric tap.

Therefore, it is an object of this invention to provide a tub from insulating material provided on the external surface with at least a bobbin, formed each by a plurality of superposed coils of lead or conductive strips to an electric tap. The connection to the tap may be obtained through a current generator pulsating at a variable frequency.

Said bobbin of lead or conductive strips, when gone through by a current, generates within the tub a pulsating electromagnetic field whose instantaneous intensity is described by the relation:

$$H = n\,I$$

wherein H is the instantaneous intensity of the magnetic field, n is the number of coils of the bobbin and I is the instantaneous intensity of the pulsating current.

The instantaneous intensity of the current and the frequency can be varied by the generator of pulsating current; there may be used frequencies variable from 1 to 5000 Hz.

The tub is from inert material, i.e. non conductive material, preferably from plastic material, such as for instance polymetacrylate or polyacrylate.

The tub may be of a simple type or provided with means suitable to form jets of hot and aerated water, so as to combine matheto-therapy and hydro-massage.

Preferably, the lead or conductive strips are from copper.

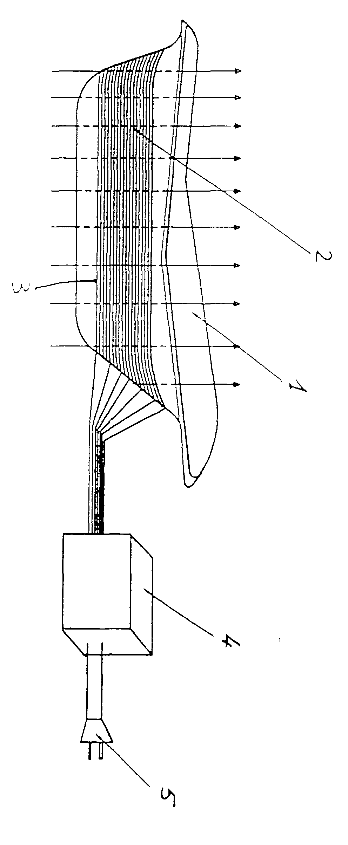

The constructive and functional characteristics of the tub of the present invention may be better understood thanks to the following detailed description wherein reference is made to the attached drawing which shows a perspective elevation view of an embodiment of the tub of the invention.

With reference to such figure, the tub comprises a container 1 from insulating material and in particular from plastic material such as polymetacrylate or polyacrylate, having, wound up on its external surface, a bobbin 2 comprised of a plurality of superposed coils 3 of lead or conductive strips, as for instance copper lead or strips.

The form and size of the tub are not critical and may be varied as one wishes, base on the requirement and the intended use, as well as the place where said tub is installed.

Tub 1 may be provided with one only bobbin 2 or a plurality of superposed bobbins, based on the surface to be covered.

The end of each bobbin 2 are connected to a generator of variable frequency pulsating current 4 which, in its turn, is connected to an electric tap through a plug 5.

Said generator 4 creates series of impulses whose frequency and intensity can be planned. The frequency may vary from 1 Hz to 5000 Hz. Besides, said generator 4 allows to store programmes of magnetic therapy and to perform automatically magnetic therapy programmes.

On the passage of electric current through the lead or the conductive strips of bobbin 2, there will generate in container 1 a magnetic field whose instantaneous intensity H is represented by the relation:

$$H = n\,I$$

wherein n is the number of coils of the bobbin and I is the instantaneous intensity of the pulsating current.

Preferably, the external surface of container 1 may be provided with longitudinal grooves, for an exact positioning of coils 3.

Even though the present invention has been described with reference to a specific embodiment of the same, it is obvious that modifications and changes may be made by those skilled in the art, following the teachings that can be inferred from the description of the present utility model.

Therefore, the present invention covers all the modifications and changes comprised within the protection scope of the appended claims.

## Claims

1. A magneto-therapic action tub, comprising a container (1) from insulating material provided on the external surface with at least a bobbin (2), connected to an electric tap; each bobbin (2) being formed by a plurality of superposed coils (3) of lead or conductive strips.

2. The tub according to claim 1, wherein the connection of bobbin (2) to an electric tap is through a variable frequency pulsating current generator (4).

3. The tub according to claim 2, wherein the frequency varieties between 1 and 5000 Hz.

4. The tub according to any of the preceding claims, wherein container (1) is from plastic material, such as polyacrylate and polymetacrylate.

5. The tub according to any of the preceding claims, wherein the lead or the conductive strip is from copper.

6. The tub according to any of the preceding claims, wherein the external surface of container (1) is provided with longitudinal grooves for an exact positioning of coils (3).

7. The tub according to any of the preceding claims, characterised in that it is provided with means for generating jets of hot and aerated water.